# EUROPÄISCHE PATENTSCHRIFT

(11) **EP 3 549 633 B1**
(45) Veröffentlichungstag und Bekanntmachung des Hinweises auf die Patenterteilung: **04.03.2026**
(21) Anmeldenummer: 19164660.3
(22) Anmeldetag: 22.03.2019
(51) Int. Cl.: A61M 35/00

(54) **MEDIZINISCHE APPLIKATIONSANORDNUNG ZUM APPLIZIEREN EINER FLÜSSIGKEIT AUF EINE HAUTFLÄCHE**
MEDICAL APPLICATION ASSEMBLY FOR APPLYING A LIQUID TO A SKIN AREA
DISPOSITIF D'APPLICATION MÉDICALE PERMETTANT D'APPLIQUER UN LIQUIDE À UNE SURFACE DE PEAU

(30) Priorität: 05.04.2018 DE 102018205105
(43) Veröffentlichungstag der Anmeldung: 09.10.2019
(73) Patentinhaber: B. Braun Melsungen AG, 34212 Melsungen (DE)
(72) Erfinder: Hofstetter, Rudolf, 6204 Sempach (CH)
(74) Vertreter: Patentanwälte Ruff, Wilhelm, Beier, Dauster & Partner mbB

(56) Entgegenhaltungen:
- WO-A1-01/14123
- WO-A1-2006/131747
- US-A1- 2003 080 151
- US-A1- 2006 072 962
- US-A1- 2012 070 220
- US-A1- 2013 108 352
- US-A1- 2014 366 485
- US-B1- 6 439 789
- US-B1- 6 729 786
- US-B2- 8 105 306

## Beschreibung

Die Erfindung betrifft eine medizinische Applikationsanordnung nach dem Oberbegriff des Anspruchs 1.

Eine derartige Applikationsanordnung ist aus der US 6 439 789 B1 bekannt, wobei deren Ampulle beispielsweise aus Glas, Aluminium, Kunststoff oder Kombinationen der genannten Materialien gefertigt sein kann. Zum Freisetzen einer in der Ampulle befindlichen Flüssigkeit weist die Ampulle ein mit einem Hohldorn durchstechbares Siegel auf. Bei einem Einstechen des Hohldorns schert das Siegel im weitesten Sinne von der Ampulle ab. Zum Dosieren der freigesetzten Flüssigkeit ist ein Dosierelement vorhanden.

Eine weitere Applikationsanordnung ist aus der US 2013/108352 A1 bekannt, wobei deren Ampulle mehrschichtig aus Kunststoff gefertigt sein kann. Zum Freisetzen einer in der Ampulle befindlichen Flüssigkeit weist die Ampulle ein Sollbruchelement auf. Bei einem Einschrauben der Ampulle in einen Aufnahmekörper wird das Sollbruchelement unter Einwirkung eines mittels eines Betätigungselements bewirkten Drehmoments wenigstens abschnittsweise von der Ampulle abgeschert. Hierdurch kann eine in der Ampulle befindliche Flüssigkeit freigesetzt werden.

Eine weitere Applikationsanordnung ist aus der US 2016/0199631 A1 bekannt und zum Applizieren eines Antiseptikums auf eine Hautfläche eines Patienten vorgesehen. Die bekannte Applikationsanordnung kann beispielsweise bei der Vorbereitung eines chirurgischen Eingriffs verwendet werden. Die bekannte Applikationsanordnung weist eine das Antiseptikum beinhaltende Ampulle auf. Die Ampulle ist in einer Aufnahmekavität eines Aufnahmekörpers festgelegt. Weiter sieht die bekannte Applikationsanordnung ein mit der Ampulle wirkverbundenes Betätigungselement vor, das hebelartig schräg von dem Aufnahmekörper abragt. Die Ampulle ist zum Freisetzen des Antiseptikums mittels manuellen Drückens des Betätigungselements zerbrechbar. Einends des Aufnahmekörpers ist ein schwammartiges Applikationselement zur Aufnahme des freigesetzten Antiseptikums vorgesehen, das in Kontakt mit der zu benetzenden Hautfläche gebracht werden kann. Bei der bekannten Applikationsanordnung ist die Ampulle vorzugsweise aus Glas gefertigt. Die Ampulle weist einen glattwandigen Zylindermantel auf, der unter Einwirkung des Betätigungselements an einer nicht näher bezeichneten Stelle bricht.

Aufgabe der Erfindung ist es, eine Applikationsanordnung der eingangs genannten Art zu schaffen, die gegenüber dem Stand der Technik verbesserte Eigenschaften aufweist und insbesondere eine verbesserte Anwendbarkeit ermöglicht.

Die der Erfindung zugrunde liegende Aufgabe wird durch eine Applikationsanordnung mit den Merkmalen des Anspruchs 1 gelöst. Durch die erfindungsgemäße Lösung ist es zum einen möglich, auf eine aus Glas gefertigte Ampulle zu verzichten. Bei solchen Glasampullen kann eine bruchbedingte Splitterbildung oftmals nicht vermieden werden. Demzufolge kann bei bekannten Applikationsanordnungen ein Filterelement zum Herausfiltern von Splittern aus der freigesetzten Flüssigkeit notwendig sein. Auf ein solches Filterelement kann erfindungsgemäß verzichtet werden, wodurch ein vereinfachter Aufbau der Applikationsanordnung ermöglicht ist. Durch die erfindungsgemäß mehrschichtig ausgebildete Wandung kann die Ampulle zudem in verbesserter Weise an spezifische Anforderungen und/oder Eigenschaften der Flüssigkeit angepasst werden. Die Wandung der Ampulle weist wenigstens eine erste und eine zweite Schicht auf. Es können jedoch auch mehr als zwei, insbesondere drei oder vier Schichten vorgesehen sein. Beispielsweise kann eine erste Schicht der Wandung hinsichtlich ihrer Stoffeigenschaften auf eine chemische Stoffzusammensetzung der Flüssigkeit abgestimmt und insbesondere sauerstoff-, kohlendioxid- und/oder wasserdampfundurchlässig ausgebildet sein. Demzufolge kann insbesondere eine verbesserte Lagerbarkeit der Flüssigkeit in der Ampulle ermöglicht werden. Beispielsweise kann eine zweite Schicht der Wandung hinsichtlich ihrer Stoffeigenschaften und/oder Dimensionierung im Hinblick auf zu erreichende mechanische Eigenschaften der Ampulle, wie insbesondere deren Festigkeit, Elastizität oder dergleichen, abgestimmt sein. Dadurch kann einem ungewollten Brechen der Ampulle entgegengewirkt werden. Dies ermöglicht letztlich eine verbesserte Anwendbarkeit der Applikationsanordnung. Die Ampulle kann mittels im Bereich der Kunststofftechnik grundsätzlich bekannter Fertigungsverfahren gefertigt sein, bspw. mittels eines Tiefzieh-, Spritzguss- oder Spritzreckblasverfahrens. Es ist möglich, dass die Ampulle zunächst mit einem einschichtigen Wandungsaufbau gefertigt wird, wobei auf diesen zunächst einschichtigen Wandungsaufbau wenigstens eine weitere Schicht, bspw. mittels eines Beschichtungsverfahrens, aufgebracht wird. Die Ampulle ist vorzugsweise formschlüssig an dem Aufnahmekörper festgelegt. Alternativ oder zusätzlich kann die Ampulle kraft- und/oder stoffschlüssig an dem Aufnahmekörper festgelegt sein. Das Betätigungselement kann beispielsweise unmittelbar an der Ampulle angeordnet, vorzugsweise an diese angeformt, oder an dem Aufnahmekörper angeordnet, vorzugsweise an diesen angeformt, sein. Das Betätigungselement kann in Form eines manuell betätigbaren Zug-, Druck-, Biege- und/oder Drehbetätigungselements ausgebildet sein. Die Ampulle kann insoweit mittels einer durch das Betätigungselement bewirkten Zug-, Druck-, Biege- und/oder Torsionsbeanspruchung zu öffnen sein. Das Betätigungselement kann eine flächen-, linienförmige oder in etwa punktuelle Beanspruchung der Ampulle bewirken. Vorzugsweise weist die Ampulle eine im Wesentlichen zylinderförmige Grundform auf. Vorzugsweise ist die Wandung der Ampulle vollständig mehrschichtig ausgebildet.

In Ausgestaltung der Erfindung weist die Wandung wenigstens eine, insbesondere Ethylen-Vinylalkohol-Copolymer (EVOH) oder Polyvinylalkohol (PVOH) beinhaltende, Barriereschicht auf. Die Barriereschicht dient in erster Linie dazu, unerwünschte Umwelteinflüsse auf die in der Ampulle eingeschlossene Flüssigkeit zu vermeiden. Hierdurch kann eine verbesserte Lagerbarkeit der Flüssigkeit ermöglicht werden. Die Barriereschicht ist vorzugsweise im Wesentlichen sauerstoff-, kohlendioxid- und/oder wasserdampfundurchlässig ausgebildet. Vorzugsweise besteht die Barriereschicht aus Ethylen-Vinylalkohol-Copolymer, dessen Barriereeigenschaften jedenfalls im Bereich der Verpackung von Nahrungsmitteln grundsätzlich bekannt sind. Alternativ kann die Barriereschicht aus Polyvinylalkohol bestehen. Die Barriereschicht kann beispielsweise extrudiert und mit einer weiteren Schicht der Wandung verbunden sein. Alternativ ist es auch möglich, dass die Barriereschicht und eine weitere Schicht der Wandung koextrudiert sind. Weiter alternativ kann die Barriereschicht beispielsweise auf eine weitere Schicht der Wandung auflaminiert sein.

In weiterer Ausgestaltung der Erfindung weist die Wandung wenigstens eine, insbesondere Polyethylen (PE) oder Polypropylen (PP) beinhaltende, Trägerschicht auf. Die Trägerschicht dient in erster Linie der Gewährleistung spezifischer mechanischer Eigenschaften der Ampulle. Beispielsweise kann die Trägerschicht derart dimensioniert sein, dass die Ampulle hinreichend robust gegenüber äußeren Transport- und/oder Lagerungseinflüssen ist, so dass ein ungewolltes Öffnen bzw. Zerbrechen der Ampulle vermieden werden kann. Die Trägerschicht kann beispielsweise extrudiert und mit einer weiteren Schicht der Wandung verbunden sein. Alternativ kann die Trägerschicht mit einer oder mehreren weiteren Schichten der Wandung koextrudiert sein. Es ist selbstverständlich auch möglich, die Trägerschicht mittels weiterer im Bereich der Kunststofftechnik üblicher Verfahren gefertigt ist und/oder mit einer weiteren oder mehreren Schichten der Wandung verbunden ist.

In weiterer Ausgestaltung der Erfindung ist die Ampulle mittels Kunststoffkoextrusion ausgebildet. Kunststoffkoextrusion ist im Bereich der Kunststofftechnik als Verfahren zur Herstellung von beispielsweise Rohren, Profilen oder dergleichen grundsätzlich bekannt. Mittels Kunststoffkoextrusion kann die mehrschichtig ausgebildete Wandung der Ampulle besonders einfach und mit gleichbleibender Qualität ausgebildet werden. Dies ist insbesondere eine besonders kostengünstige Ausgestaltung der Erfindung.

In weiterer Ausgestaltung der Erfindung ist die Ampulle mittels eines Blow-Fill-Seal-Verfahrens aseptisch ausgebildet. Blow-Fill-Seal-Verfahren sind im Bereich der Kunststoffverarbeitung grundsätzlich bekannt und bilden eine Sonderform des Extrusionsblasformens. Dabei wird aufgeschmolzener Kunststoff mittels einer Extruderschnecke durch eine kreisringförmige Düse gepresst, so dass ein schlauchförmiges Halbzeug entsteht. Dieses wird in eine Blasform übergeben und mittels Innendruck einer Innenkontur der Blasform angepasst. Der Innendruck wird mittels Injektionsnadeln aufgebracht, wobei gleichzeitig die Flüssigkeit, beispielsweise ein Antiseptikum, injiziert wird. Die Injektionsnadeln werden hiernach aus dem aufgeblasenen Halbzeug zurückgezogen und der Formprozess auf grundsätzlich bekannte Weise abgeschlossen. Das Blow-Fill-Seal-Verfahren findet in einem sterilen Abschnitt einer Kunststoffverarbeitungsmaschine statt. Durch diese Ausgestaltung der Erfindung können erforderliche aseptische Eigenschaften der Ampulle auf besonders einfache und zuverlässige Weise gewährleistet werden.

In weiterer Ausgestaltung der Erfindung weist die Ampulle wenigstens einen elastisch nachgiebig gestalteten Wandungsabschnitt auf. Vorzugsweise weist die Ampulle eine zylinderförmige Grundform auf. Der Wandungsabschnitt ist vorzugsweise in radialer Richtung elastisch nachgiebig gestaltet. So kann nach Öffnen der Ampulle mittels manueller Druckausübung auf den Wandungsabschnitt ein Volumenstrom der Flüssigkeit aus der Ampulle beeinflusst werden. Dadurch kann insbesondere eine verbesserte Dosierbarkeit der Flüssigkeit ermöglicht werden. Durch diese Ausgestaltung der Erfindung wird eine besonders einfach handhabbare Applikationsanordnung erreicht.

In weiterer Ausgestaltung der Erfindung sind der Aufnahmekörper, das Applikationselement und das Betätigungselement aus Kunststoff gefertigt. Da auch die Ampulle erfindungsgemäß aus Kunststoff gefertigt ist, erlaubt diese Ausgestaltung der Erfindung eine vereinfachte und umweltfreundliche Entsorgung der Applikationsanordnung. Vorzugsweise ist die gesamte Applikationsanordnung ausschließlich aus Kunststoff gefertigt.

Weiter gemäß der Erfindung ist ein der Ampulle und/oder dem Applikationselement zugeordnetes Dosierelement vorgesehen, mittels dessen ein Volumenstrom von aus der Ampulle freigesetzter Flüssigkeit dosierbar ist. Durch die erfindungsgemäße Lösung kann eine verbesserte Dosierbarkeit der nach Öffnen der Ampulle freigesetzten Flüssigkeit erreicht werden. Das Dosierelement ist vorzugsweise derart gestaltet, dass jedenfalls einem ungehinderten Abfließen der freigesetzten Flüssigkeit aus der Ampulle entgegengewirkt ist. Zu diesem Zweck kann das Dosierelement beispielsweise eine Verengung eines fluidführenden Lumens der Applikationsanordnung, beispielsweise eines Lumens des Aufnahmekörpers oder des Applikationselements, bilden oder selbst ein fluidführendes Lumen aufweisen, das mit einer Verengung versehen ist. Dadurch kann insbesondere eine Überdosierung der Flüssigkeit vermieden werden. Auf diese Weise kann eine verbesserte Anwendbarkeit und insbesondere eine verbesserte Anwendungssicherheit erreicht werden. Letzteres, da insbesondere eine übermäßige Benetzung der zu behandelnden Hautfläche mit Flüssigkeit vermieden werden kann. Das Dosierelement kann form-, kraft- und/oder stoffschlüssig an dem Aufnahmekörper festgelegt sein. Vorzugsweise ist das Dosierelement in einem fluidführenden Durchlass oder Lumen des Aufnahmekörpers angeordnet.

Weiter gemäß der Erfindung ist das Dosierelement in Abhängigkeit eines Flüssigkeitsdrucks der Flüssigkeit zwischen wenigstens einer ersten Dosierstellung und einer zweiten Dosierstellung überführbar. Der Flüssigkeitsdruck kann beispielsweise mittels einer manuellen Druckeinwirkung auf die Ampulle bewirkt sein. Vorzugsweise wird das Dosierelement mittels des Flüssigkeitsdrucks deformiert und somit zwischen der ersten Dosierstellung und der zweiten Dosierstellung verlagert. Vorzugsweise bildet die erste Dosierstellung eine Schließstellung, in der ein Volumenstrom der freigesetzten Flüssigkeit im Wesentlichen vollständig unterbunden ist. Vorzugsweise bildet die zweite Dosierstellung eine Öffnungsstellung, in der ein Volumenstrom der freigesetzten Flüssigkeit freigegeben ist. Es ist vorteilhaft, wenn das Dosierelement kontinuierlich zwischen der ersten Dosierstellung und der zweiten Dosierstellung verlagerbar ist. Auf diese Weise kann eine stufenlose Dosierung der Flüssigkeit ermöglicht werden.

In weiterer Ausgestaltung der Erfindung weist das Dosierelement eine gummielastische Membran auf, die druckabhängig permeabel ausgebildet ist. Die gummielastische Membran ist vorzugsweise in Form einer kreiszylinderförmigen Tellermembran ausgebildet. Um eine druckabhängige Permeabilität der Membran zu erreichen, kann diese wenigstens einen Fluiddurchlass aufweisen, der in Abhängigkeit des Fluiddrucks der Flüssigkeit geöffnet, geschlossen, verengt und/oder aufgeweitet werden kann. Die Membran kann beispielsweise aus einem natürlich oder synthetisch hergestellten Elastomer, einem thermoplastischen Elastomer, Silikon oder dergleichen gefertigt sein.

In weiterer Ausgestaltung der Erfindung weist die Membran wenigstens einen Schlitz auf. Der Schlitz bildet einen Fluiddurchlass für die aus der Ampulle freigesetzte Flüssigkeit. Der Schlitz ist vorzugsweise derart ausgebildet und/oder angeordnet, dass mittels einer fluiddruckbedingten Auslenkung der Membran ein Öffnen und/oder Schließen des Schlitzes bewirkt ist.

Weiter gemäß der Erfindung weist die Ampulle ein mit dem Betätigungselement wirkverbundenes Sollbruchelement auf. Das Sollbruchelement bildet eine Sollbruchstelle und/oder einen Sollbruchabschnitt der Ampulle. Das Sollbruchelement ist derart gestaltet, dass es bei einer bestimmungsgemäßen Betätigung des Betätigungselements unter einer konstruktiv vorherbestimmten Beanspruchung gezielt versagt. Durch die erfindungsgemäße Lösung kann demnach ein örtlich undefiniertes Zerbrechen der Ampulle vermieden und somit ein verbessertes Öffnen der Ampulle ermöglicht werden. Hierdurch kann eine verbesserte Freisetzung der Flüssigkeit und letztlich eine verbesserte Anwendbarkeit der Applikationsanordnung erreicht werden.

In weiterer Ausgestaltung der Erfindung ist das Sollbruchelement duktil brechbar gestaltet. Demnach versagt das Sollbruchelement unter Beanspruchung nicht unter Ausbildung eines Sprödbruchs, sondern unter Ausbildung eines Verformungsbruchs. Dadurch kann einer Splitterbildung beim Öffnen der Ampulle entgegengewirkt werden. Zudem kann eine verbesserte haptische Rückmeldung bei der Betätigung des Betätigungselements erreicht werden, da die Ampulle nicht abrupt spröde bricht, sondern zunächst ein gewisser Verformungsweg und damit Betätigungsweg des Betätigungselementes erforderlich ist.

Weiter gemäß der Erfindung ist das Betätigungselement drehmomentübertragend mit dem Sollbruchelement wirkverbunden, wobei die Ampulle derart ausgebildet ist, dass das Sollbruchelement unter Einwirkung einer bestimmten mittels des Betätigungselements bewirkten Drehmomentbeanspruchung wenigstens abschnittsweise von der Ampulle abschert. Das Betätigungselement kann mittelbar oder unmittelbar drehmomentübertragend mit dem Sollbruchelement wirkverbunden sein. Es hat sich gezeigt, dass der derart erreichte Drehmechanismus zur Öffnung der Ampulle insbesondere ergonomisch vorteilhaft ist.

Weiter gemäß der Erfindung ist das Sollbruchelement an einem distalen Ende der Ampulle in Form eines Knebels ausgebildet. Optional ist das Betätigungselement, vorzugsweise an einem proximalen Ende der Ampulle, in Form eines Drehbetätigungselements ausgebildet. Unter dem distalen Ende der Ampulle ist dasjenige Ende zu verstehen, das bei einem bestimmungsgemäßen Gebrauch der Applikationsanordnung der zu benetzenden Hautfläche zugewandt ist. Demgemäß ist unter dem proximalen Ende der Ampulle dasjenige Ende zu verstehen, das der Hautfläche abgewandt ist. Der Knebel und das Drehbetätigungselement sind vorzugsweise koaxial zueinander angeordnet. Der Knebel ist formschlüssig an einem komplementär ausgebildeten Aufnahmeabschnitt des Aufnahmekörpers festgelegt. Das Drehbetätigungselement kann unmittelbar mit dem proximalen Ende der Ampulle verbunden und beispielsweise in Form eines Mantelabschnitts der Ampulle ausgebildet sein. Um eine leichtere Handhabung zu ermöglichen, kann das Drehbetätigungselement in diesem Fall flügelartig, ähnlich wie bei einer Flügelmutter, gestaltet sein. Alternativ ist es auch möglich, dass das Drehbetätigungselement eine Art Stopfen bildet, mittels dessen die Ampulle an ihrem proximalen Ende fluiddicht verschlossen ist. Es ist jedoch auch möglich, dass das Drehbetätigungselement ein separat von der Ampulle gefertigtes und mit dieser verbundenes Bauteil ist. Beispielsweise kann das Drehbetätigungselement mittels einer Steck-, Schraub-, Kleb- oder anderweitigen Verbindung mit dem proximalen Ende der Ampulle verbunden sein. Vorzugsweise ist das Drehbetätigungselement in Form eines Deckels stirnendseitig auf die Ampulle und/oder den Aufnahmekörper aufgesteckt.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in einer schematischen teilweise aufgeschnittenen Seitenansicht eine Ausführungsform einer erfindungsgemäßen Applikationsanordnung, die eine aus Kunststoff gefertigte Ampulle aufweist,
- Fig. 2: in einer schematischen teilweise aufgeschnittenen Seitenansicht die Ampulle der Applikationsanordnung nach Fig. 1,
- Fig. 3: in einer vergrößerten Detaildarstellung eine Aufsicht eines Dosierelements der Applikationsanordnung nach Fig. 1,
- Fig. 4: das Dosierelement nach Fig. 3 in einem schematischen Querschnitt IV und
- Fig. 5: in einer vergrößerten schematischen Detaildarstellung eine Wandung der Ampulle nach Fig. 2 in einem Bereich V gemäß Fig. 2.

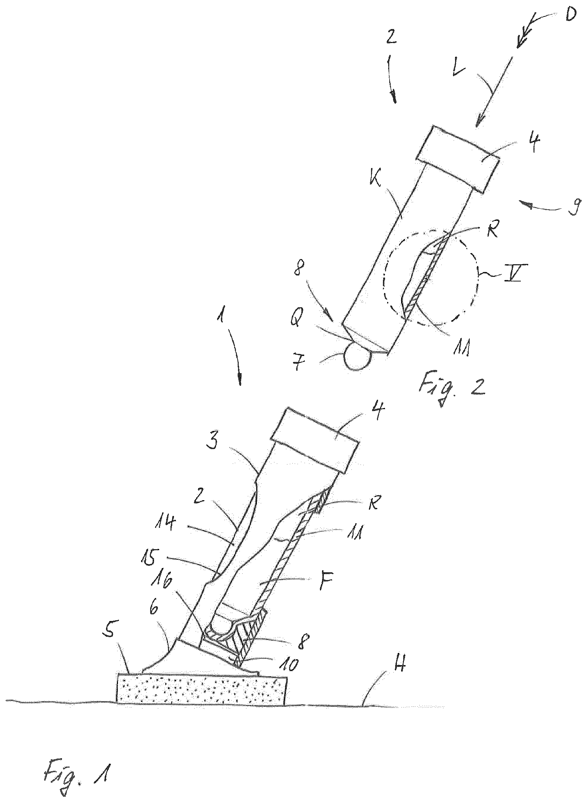

Eine medizinische Applikationsanordnung 1 gemäß Fig. 1 ist zum Applizieren einer Flüssigkeit F auf eine Hautfläche H vorgesehen. Die Applikationsanordnung 1 weist eine die Flüssigkeit F beinhaltende Ampulle 2 auf, die an einem Aufnahmekörper 3 festgelegt ist. Wie insbesondere anhand Fig. 2 ersichtlich ist, weist die Ampulle 2 eine im Wesentlichen zylinderförmige Grundform auf. Die Ampulle bildet eine fluiddichte Kavität R aus, in der die Flüssigkeit F aufgenommen ist. Selbstverständlich ist es auch möglich, dass die Ampulle mehrere fluiddichte Kavitäten ausbildet, in denen jeweils eine Flüssigkeit aufgenommen ist. Auf diese Weise kann die Ampulle ein Mehrkammersystem bilden, das die Applikation und/oder ein Mischen mehrerer unterschiedlicher Flüssigkeiten ermöglicht.

Der Aufnahmekörper 3 ist vorliegend in Form eines längserstreckten Hohlzylinders ausgebildet. In einem bestimmungsgemäß zusammengefügten Zustand der Applikationsanordnung 1 (Fig. 1) ist die Ampulle 2 durch eine nicht näher ersichtliche proximale Öffnung des Aufnahmekörpers 3 in diesen eingesteckt und auf noch näher beschriebene Weise an diesem festgelegt. Die Applikationsanordnung 1 weist weiter ein mit der Ampulle 2 wirkverbundenes Betätigungselement 4 auf. Das Betätigungselement 4 dient, wie nachfolgend noch näher beschrieben wird, zum Öffnen der Ampulle 2 und zum Freisetzen der Flüssigkeit F. Das Betätigungselement 4 ist vorliegend an einem proximalen Ende des Aufnahmekörpers 3 angeordnet. Weiter ist an einem dem Betätigungselement 4 abgewandten Stirnendbereich des Aufnahmekörpers 3 ein Applikationselement 5 angeordnet. Das Applikationselement 5 ist zum Applizieren von aus der Ampulle 2 freigesetzter Flüssigkeit F vorgesehen und vorliegend in Form eines aus einem Kunststoffmaterial gefertigten Schwamms ausgebildet. Bei einer nicht näher dargestellten Ausführungsform ist anstelle eines Schwamms ein Mikrofasertuch aus PET vorgesehen. Alternativ ist es auch möglich, das Applikationselement nach Art eines Tupfers auszuführen. Grundsätzlich kann das Applikationselement saugfähig oder nicht saugfähig ausgebildet sein. Vorliegend ist in Axialrichtung des Aufnahmekörpers 3 zwischen dem Applikationselement 5 und dem Aufnahmekörper 3 ein in etwa keilförmig ausgebildetes Verbindungselement 6 vorgesehen, was jedoch nicht zwingend erforderlich ist. Das Verbindungselement 6 schafft eine feste Verbindung zwischen dem Applikationselement 5 und dem Aufnahmekörper 3, so dass letzterer schräg nach oben nach Art eines Stiels von dem Applikationselement 5 abragt. Bei einer nicht dargestellten Ausführungsform ist kein Verbindungselement vorgesehen, so dass das Applikationselement in etwa senkrecht zu dem Aufnahmekörper orientiert ist, was eine senkrechte Applikation auf der zu benetzenden Fläche ermöglicht.

Wie insbesondere anhand Fig. 2 ersichtlich ist, weist die Ampulle 2 ein mit dem Betätigungselement 4 wirkverbundenes Sollbruchelement 7 auf. Das Sollbruchelement 7 ist an einem distalen Ende 8 der Ampulle 2 angeordnet. Das Betätigungselement 4 ist an einem proximalen Ende 9 der Ampulle 2 angeordnet. Dabei ist das Betätigungselement 4 vorliegend fest an die Ampulle 2 angeformt und insoweit einstückig mit dieser verbunden, was jedoch nicht zwingend der Fall sein muss. Bei einer nicht dargestellten Ausführungsform kann das Betätigungselement 4 auch separat von der Ampulle 2 gefertigt und mit dem proximalen Ende 9 der Ampulle wirkverbunden sein. Hierbei kann das Betätigungselement 4 nach Art eine Deckels stirnendseitig auf die Ampulle 2 und/oder stirnendseitig auf den Aufnahmekörper 3 aufgesteckt sein.

Das Sollbruchelement ist in Form eines Knebels 7 ausgebildet, der einstückig an die Ampulle 2 angeformt ist. Der Knebel 7 bildet eine stirnendseitige Verjüngung des Querschnitts der Ampulle 2, die ansonsten glattwandig ausgebildet ist. Wie anhand Fig. 1 ersichtlich ist, ist der Knebel 7 in einem bestimmungsgemäß zusammengesetzten Zustand der Applikationsanordnung 1 drehfest an einem komplementären Aufnahmeabschnitt 8 des Aufnahmekörpers 3 festgelegt. Der Knebel 7 ist in Umfangsrichtung der Ampulle 2 formschlüssig mit dem Aufnahmeabschnitt 8 zusammengefügt, so dass die Ampulle 2 - jedenfalls in einem intakten Zustand des Sollbruchelements 7 - im Bereich des Sollbruchelements 7 drehmomentfest mit dem Aufnahmekörper 3 verbunden ist. Ansonsten ist jedenfalls in Umfangsrichtung keine feste Verbindung zwischen der Ampulle 2 und dem Aufnahmekörper 3 vorgesehen.

Die Ampulle 2 ist derart ausgebildet, dass das Sollbruchelement 7 unter Einwirkung einer bestimmten mittels des Betätigungselements 4 bewirkten Drehmomentbeanspruchung wenigstens abschnittsweise von der Ampulle 2 abschert. Zu diesem Zweck ist das Betätigungselement drehmomentübertragend mit dem Sollbruchelement 4 verbunden und in Form eines Drehbetätigungselements 4 ausgebildet.

Bei einer nicht erfindungsgemäßen Ausführungsform kann das Sollbruchelement in Form eines Einstechabschnitts ausgebildet sein, dem ein Einstechdorn zum Einstechen in den Einstechabschnitt und damit zum Freisetzen der Flüssigkeit F zugeordnet sein kann.

Zum Applizieren der Flüssigkeit F auf der Hautfläche H wird die Applikationsanordnung 1 in der anhand Fig. 1 ersichtlichen Orientierung gegenüber der Hautfläche H ausgerichtet. Hierzu umgreift eine nicht näher ersichtliche Bedienperson den stielartigen Bereich des Aufnahmekörpers 3 und setzt das Applikationselement 5 auf die zu benetzende Hautfläche H auf. In diesem Zustand ist die Ampulle 2 fluiddicht verschlossen und die Flüssigkeit F in der Kavität R angeordnet. Zum Öffnen der Ampulle 2 und damit zum Freisetzen der Flüssigkeit F umgreift die Bedienperson das Betätigungselement 4 vorzugsweise mit Daumen und Zeigefinger und bringt ein entlang einer Längsachse L der Ampulle 2 wirkendes Drehmoment D auf. Da das Betätigungselement 4 drehmomentübertragend mit dem Sollbruchelement 7 wirkverbunden und dieses drehfest in dem Aufnahmeabschnitt 8 festgelegt ist, wird das Sollbruchelement 7 hierbei drehmomentbeansprucht. Dabei ist das Sollbruchelement 7 bzw. ein an das Sollbruchelement 7 angrenzender Querschnitt Q der Ampulle 2 derart dimensioniert, dass das Sollbruchelement 7 bei einer vorherbestimmten, definierten Drehmomentbeanspruchung wenigstens abschnittsweise von der Ampulle 2 abschert. Hierdurch wird die Ampulle 2 geöffnet und die Flüssigkeit F kann durch eine im Bereich des Querschnitts Q ausgebildete Versagensstelle aus der Ampulle 2 ausfließen. Schwerkraftbedingt fließt die Flüssigkeit F - in Bezug auf die Zeichenebene der Fig. 1 - nach unten und gelangt unter anderem über einen Durchlass 10 des Aufnahmekörpers 3 und einen nicht näher ersichtlichen Durchlass des Verbindungselements 6 zu dem Applikationselement 5. Das Applikationselement 5 nimmt die Flüssigkeit F schwammartig auf. Die Bedienperson führt hiernach die Applikationsanordnung manuell und an dem stielartigen Abschnitt des Aufnahmekörpers 3 entlang der Hautfläche H und appliziert somit die Flüssigkeit F. Bei einer nicht näher dargestellten Ausführungsform ist vorgesehen, dass ein mit einem Zusatzstoff getränkter fluiddurchlässiger Körper in dem Durchlass 10 angeordnet ist. Auf diese Weise kann der Flüssigkeit beim Durchströmen des Durchlasses der Zusatzstoff zudosiert werden. Bei diesem Zusatzstoff kann es sich um einen Farbstoff, einen Stoff mit sporozider Wirkung oder dergleichen handeln.

Das Sollbruchelement 7 ist vorliegend duktil brechbar gestaltet und schert insoweit nicht spröde, sondern erst nach einer gewissen plastischen Deformation von der Ampulle ab. Die derartige Gestaltung des Sollbruchelements 7 kann mittels einer entsprechenden Werkstoffwahl der Ampulle 2 erreicht werden.

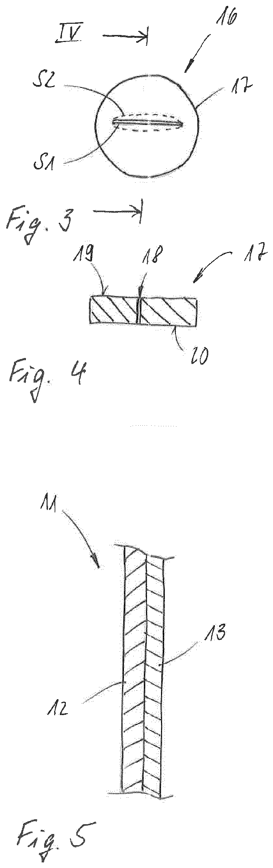

Die Ampulle 2 ist aus Kunststoff K gefertigt. Dabei sind die Stoffeigenschaften des Kunststoffs K vorliegend derart gewählt, dass das Sollbruchelement 7 unter Beanspruchung mit dem Drehmoment D auf die vorbeschriebene Weise duktil bricht. Dadurch kann eine Splitterbildung beim Öffnen der Ampulle 2 vermieden werden. Wie weiter anhand Fig. 2 in Verbindung mit Fig. 5 ersichtlich ist, weist die Ampulle 2 eine mehrschichtig ausgebildete Wandung 11 auf. Die Wandung 11 weist wenigstens eine Barriereschicht 12 auf. Die Barriereschicht 12 dient dem Schutz der Flüssigkeit F vor unerwünschten Umwelteinflüssen und kann insbesondere sauerstoff-, kohlendioxid- und/oder wasserdampfundurchlässig gestaltet sein. Vorliegend besteht die Barriereschicht 12 aus Ethylen-Vinylalkohol-Copolymer. Bei einer alternativen Ausführungsform kann die Barriereschicht 12 beispielsweise aus Polyvinylalkohol bestehen. Die Barriereschicht 12 bildet vorliegend eine Innenoberfläche der Ampulle 2 aus und steht insoweit in unmittelbarem Kontakt mit der Flüssigkeit F.

Wie weiter anhand Fig. 5 ersichtlich ist, weist die Wandung 12 wenigstens eine Trägerschicht 13 auf. Die Trägerschicht 13 dient in erster Linie der Gewährleistung der mechanischen Eigenschaften der Ampulle 2. Die Trägerschicht 13 besteht vorliegend aus Polyethylen. Bei einer alternativen Ausführungsform besteht die Trägerschicht 13 aus Polypropylen. Vorliegend bildet die Trägerschicht 13 eine Außenfläche der Ampulle 2. Die Ampulle 2 weist somit einen zweischichtig ausgebildeten Wandungsaufbau auf. Grundsätzlich können auch mehr als zwei Schichten vorgesehen sein.

Die Ampulle 2, genauer die Wandung 11, ist mittels Kunststoffkoextrusion ausgebildet. Dabei wird sowohl der Kunststoffwerkstoff der Barriereschicht 12 als auch der Kunststoffwerkstoff der Trägerschicht 13 erweicht und mittels einer Extruderschnecke auf grundsätzlich bekannte Weise gemeinsam durch eine Düse gepresst. Zur aseptischen Ausbildung der Ampulle 2 findet vorliegend ein sogenanntes Blow-Fill-Seal-Verfahren Anwendung, das als Sonderform der Blasextrusion im Bereich der Kunststoffverarbeitung grundsätzlich bekannt ist.

Die Ampulle 2 weist wenigstens einen elastisch nachgiebig gestalteten Wandungsabschnitt 14 auf (Fig. 1). Der Wandungsabschnitt 14 ist in Längsrichtung L der Ampulle 2 in etwa mittig angeordnet und in radialer Richtung nachgiebig gestaltet. In dem anhand Fig. 1 ersichtlichen bestimmungsgemäß zusammengesetzten Zustand der Applikationsanordnung 1 ist der Wandungsabschnitts 14 im Bereich einer Ausnehmung 15 des Aufnahmekörpers 3 angeordnet. Demnach ist der Wandungsabschnitt 14 manuell zugänglich und kann von der Bedienperson, beispielsweise mittels eines im Bereich der Ausnehmung 15 angeordneten Daumens, gedrückt werden. Nach Öffnen der Ampulle 2 kann auf diese Weise mittels Drückens des Wandungsabschnitts 14 das Ausfließen der Flüssigkeit F beeinflusst werden.

Vorliegend sind neben der Ampulle 2 auch der Aufnahmekörper 3, das Betätigungselement 4, das Applikationselement 5 und das Verbindungselement 6 aus Kunststoff gefertigt. Insoweit ist die gesamte Applikationsanordnung 1 aus Kunststoff gefertigt, was eine Entsorgung nach einer einmaligen Anwendung erleichtern und gegebenenfalls umweltfreundlich gestalten kann.

Wie weiter anhand Fig. 1 ersichtlich ist, ist ein der Ampulle 2 und/oder dem Applikationselement 5 zugeordnetes Dosierelement 16 vorgesehen, mittels dessen ein Volumenstrom von aus der Ampulle 2 freigesetzter Flüssigkeit F dosierbar ist. Das Dosierelement 16 ist vorliegend in Längsrichtung L der Ampulle 2 zwischen dem Sollbruchelement 7 und dem Durchlass 10 des Aufnahmekörpers 3 angeordnet und auf nicht näher dargestellte Weise randseitig im Bereich des Durchlasses 10 an dem Aufnahmekörper 3 festgelegt. Wie anhand der Fig. 3, 4 ersichtlich ist, weist das Dosierelement 16 eine Membran 17 auf. Die Membran 17 ist mit einem Schlitz 18 versehen. Der Schlitz 18 ist zwischen einer Oberseite 19 und einer Unterseite 20 der Membran 17 im Wesentlichen parallel zur Längsrichtung L der Ampulle 2 längserstreckt. In radialer Richtung erstreckt sich der Schlitz 18 über in etwa zwei Drittel des Durchmessers der Membran 17, die vorliegend tellerförmig gestaltet ist und insoweit eine kreiszylindrische Grundform aufweist. Anhand Fig. 4 ist eine Dickenabmessung der Membran 17 allein aus zeichnerischen Gründen stark überhöht dargestellt. Entgegen der vereinfachten zeichnerischen Darstellung der Fig. 4 ist die Membran 17 tatsächlich dünnwandig gestaltet. Infolge der derartigen Gestaltung ist die Membran 17 in Abhängigkeit eines Flüssigkeitsdrucks der Flüssigkeit F zwischen wenigstens einer ersten Dosierstellung S1 und einer zweiten Dosierstellung S2 überführbar. Diese beiden Dosierstellungen S1, S2 sind schematisch anhand Fig. 3 angedeutet. In der ersten Dosierstellung S1 sind in radialer Richtung gegenüberliegend angeordnete nicht näher bezeichnete Seitenwandungen des Schlitzes 18 einander kontaktierend angeordnet, so dass der Durchlass von der Oberseite 19 zu der Unterseite 20 der Membran 17 im Wesentlichen verschlossen ist. Bei einer Druckbeaufschlagung der Membran 17, die beispielsweise mittels einer manuellen Druckbetätigung des Wandungsabschnitts 14 der Ampulle bewirkt sein kann, wird die Membran 17 deformiert. Der derart bewirkte deformierte Zustand der Membran 17 ist anhand Fig. 3 mittels der dortigen strichlierten Linie verdeutlicht. Dabei ist der Schlitz 18 in radialer Richtung aufgeweitet, so dass die Seitenwandungen des Schlitzes 18 voneinander beabstandet sind. Hierdurch wird der Durchlass von der Oberseite 19 zu der Unterseite 20 der Membran 17 freigegeben und bereits freigesetzte Flüssigkeit F kann durch den Schlitz 18 in den Durchlass 10 des Aufnahmekörpers 3 und von dort weiter zu dem Applikationselement 5 gelangen.

## Patentansprüche

1. Medizinische Applikationsanordnung (1) zum Applizieren einer Flüssigkeit (F) auf eine Hautfläche (H) mit einer die Flüssigkeit (F) beinhaltenden Ampulle (2), einem Aufnahmekörper (3), an dem die Ampulle (2) festgelegt ist, einem mit der Ampulle (2) wirkverbundenen Betätigungselement (4) zum Öffnen der Ampulle (2) und Freisetzen der Flüssigkeit (F) und einem einends des Aufnahmekörpers (3) angeordneten Applikationselement (5) zum Applizieren von aus der Ampulle (2) freigesetzter Flüssigkeit (F), wobei ein der Ampulle (2) und/oder dem Applikationselement (5) zugeordnetes Dosierelement (16) vorgesehen ist, mittels dessen ein Volumenstrom von aus der Ampulle (2) freigesetzter Flüssigkeit (F) dosierbar ist, wobei das Dosierelement (16) in Abhängigkeit eines Flüssigkeitsdrucks der Flüssigkeit (F) zwischen wenigstens einer ersten Dosierstellung (S1) und einer zweiten Dosierstellung (S2) überführbar ist, wobei die Ampulle (2) ein mit dem Betätigungselement (4) wirkverbundenes Sollbruchelement (7) aufweist, wobei das Betätigungselement (4) drehmomentübertragend mit dem Sollbruchelement (7) wirkverbunden ist, und wobei die Ampulle (2) derart ausgebildet ist, dass das Sollbruchelement (7) unter Einwirkung einer bestimmten mittels des Betätigungselements (4) bewirkten Drehmomentbeanspruchung wenigstens abschnittsweise von der Ampulle (2) abschert,
**dadurch gekennzeichnet, dass** die Ampulle (2) aus Kunststoff (K) gefertigt ist und eine wenigstens abschnittsweise mehrschichtig ausgebildete Wandung (11) aufweist, und dass das Sollbruchelement an einem distalen Ende (8) der Ampulle (2) in Form eines Knebels (7) einstückig an die Ampulle (2) angeformt ist, wobei der Knebel (7) eine stirnendseitige Verjüngung eines Querschnitts der Ampulle (2) bildet, und wobei der Knebel (7) in Umfangsrichtung der Ampulle (2) formschlüssig mit einem komplementären Aufnahmeabschnitt (8) des Aufnahmekörpers (3) zusammengefügt ist, so dass die Ampulle (2) - jedenfalls in einem intakten Zustand des Knebels (7) - im Bereich des Knebels (7) drehmomentfest mit dem Aufnahmekörper (3) verbunden ist.

2. Medizinische Applikationsanordnung (1) nach Anspruch 1, **dadurch gekennzeichnet, dass** die Wandung (11) wenigstens eine, insbesondere Ethylen-Vinylalkohol-Copolymer oder Polyvinylalkohol beinhaltende, Barriereschicht (12) aufweist.

3. Medizinische Applikationsanordnung (1) nach Anspruch 1 oder 2, **dadurch gekennzeichnet, dass** die Wandung (11) wenigstens eine, insbesondere Polyethylen oder Polypropylen beinhaltende, Trägerschicht (13) aufweist.

4. Medizinische Applikationsanordnung (1) nach einem der vorhergehenden Ansprüche, **dadurch gekennzeichnet, dass** die Ampulle (2) mittels Kunststoffkoextrusion ausgebildet ist.

5. Medizinische Applikationsanordnung (1) nach einem der vorhergehenden Ansprüche, **dadurch gekennzeichnet, dass** die Ampulle (2) mittels eines Blow-Fill-Seal-Verfahrens aseptisch ausgebildet ist.

6. Medizinische Applikationsanordnung (1) nach einem der vorhergehenden Ansprüche, **dadurch gekennzeichnet, dass** die Ampulle (2) wenigstens einen elastisch nachgiebig gestalteten Wandungsabschnitt (14) aufweist.

7. Medizinische Applikationsanordnung (1) nach einem der vorhergehenden Ansprüche, **dadurch gekennzeichnet, dass** der Aufnahmekörper (3), das Applikationselement (5) und das Betätigungselement (4) aus Kunststoff gefertigt sind.

8. Medizinische Applikationsanordnung (1) nach einem der vorhergehenden Ansprüche, **dadurch gekennzeichnet, dass** das Dosierelement (16) eine gummielastische Membran (17) aufweist, die druckabhängig permeabel ausgebildet ist.

9. Medizinische Applikationsanordnung (1) nach Anspruch 8, **dadurch gekennzeichnet, dass** die Membran (17) wenigstens einen Schlitz (18) aufweist.

10. Medizinische Applikationsanordnung (1) nach einem der vorhergehenden Ansprüche, **dadurch gekennzeichnet, dass** der Knebel (7) duktil brechbar gestaltet ist.

11. Medizinische Applikationsanordnung (1) nach einem der vorhergehenden Ansprüche, **dadurch gekennzeichnet, dass** das Betätigungselement, vorzugsweise an einem proximalen Ende (9) der Ampulle (2), in Form eines Drehbetätigungselements (4) ausgebildet ist.

## Claims

1. Medical applicator (1) for applying a liquid (F) to a skin surface (H), comprising an ampoule (2) containing the liquid (F), a receiving body (3) to which the ampoule (2) is secured, an actuation element (4) operatively connected to the ampoule (2) for opening the ampoule (2) and releasing the liquid (F), and an application element (5) arranged at one end of the receiving body (3) for applying liquid (F) released from the ampoule (2), wherein a dosing element (16) assigned to the ampoule (2) and/or to the application element (5) is provided, by means of which dosing element a volumetric flow of liquid (F) released from the ampoule (2) can be dosed, wherein the dosing element (16) can be transferred between at least a first dosing position (S1) and a second dosing position (S2) depending on a liquid pressure of the liquid (F), wherein the ampoule (2) has a predetermined breaking element (7) operatively connected to the actuation element (4), wherein the actuation element (4) is operatively connected to the predetermined breaking element (7) in a torque-transmitting manner, and wherein the ampoule (2) is designed in such a way that the predetermined breaking element (7) shears away from the ampoule (2) at least in sections under the effect of a specific torque load brought about by means of the actuation element (4),
**characterized in that** the ampoule (2) is produced from plastic (K) and has a wall (11) which is of a multi-layer design at least in sections, and **in that** the predetermined breaking element is integrally formed on the ampoule (2), in the form of a toggle (7) at a distal end (8) of the ampoule (2), wherein the toggle (7) forms an end-side tapering of a cross section of the ampoule (2), and wherein the toggle (7) is joined in a form-fitting manner in the circumferential direction of the ampoule (2) to a complementary receiving portion (8) of the receiving body (3), such that the ampoule (2) - at any rate in an intact state of the toggle (7) - is connected to the receiving body (3) fixedly in terms of torque in the region of the toggle (7).

2. Medical applicator (1) according to Claim 1, **characterized in that** the wall (11) has at least one barrier layer (12), in particular comprising ethylenevinyl alcohol copolymer or polyvinyl alcohol.

3. Medical applicator (1) according to Claim 1 or 2, **characterized in that** the wall (11) has at least one carrier layer (13), in particular comprising polyethylene or polypropylene.

4. Medical applicator (1) according to any one of the preceding claims, **characterized in that** the ampoule (2) is formed by means of plastic coextrusion.

5. Medical applicator (1) according to any one of the preceding claims, **characterized in that** the ampoule (2) is formed aseptically by means of a blow-fill-seal process.

6. Medical applicator (1) according to any one of the preceding claims, **characterized in that** the ampoule (2) has at least one elastically flexible wall portion (14).

7. Medical applicator (1) according to any one of the preceding claims, **characterized in that** the receiving body (3), the application element (5) and the actuation element (4) are produced from plastic.

8. Medical applicator (1) according to any one of the preceding claims, **characterized in that** the dosing element (16) has a rubber-elastic membrane (17) which is designed to be permeable in a pressure-dependent manner.

9. Medical applicator (1) according to Claim 8, **characterized in that** the membrane (17) has at least one slit (18).

10. Medical applicator (1) according to any one of the preceding claims, **characterized in that** the toggle (7) is designed to be breakable with a ductile fracture.

11. Medical applicator (1) according to any one of the preceding claims, **characterized in that** the actuation element, preferably at a proximal end (9) of the ampoule (2), is in the form of a rotary actuation element (4).

## Revendications

1. Agencement d'application médical (1) pour appliquer un liquide (F) sur une surface cutanée (H), avec une ampoule (2) contenant le liquide (F), un corps de réception (3) sur lequel est fixée l'ampoule (2), un élément d'actionnement (4) relié de manière fonctionnelle à l'ampoule (2) pour ouvrir l'ampoule (2) et libérer le liquide (F), et un élément d'application (5) agencé à une extrémité du corps de réception (3) pour appliquer le liquide (F) libéré de l'ampoule (2), un élément de dosage (16) associé à l'ampoule (2) et/ou à l'élément d'application (5) étant prévu, au moyen duquel un débit volumique du liquide (F) libéré de l'ampoule (2) peut être dosé, l'élément de dosage (16) pouvant être transféré entre au moins une première position de dosage (S1) et une deuxième position de dosage (S2) en fonction d'une pression de liquide du liquide (F), l'ampoule (2) présentant un élément de rupture prédéterminée (7) relié de manière fonctionnelle à l'élément d'actionnement (4), l'élément d'actionnement (4) étant relié de manière fonctionnelle à l'élément de rupture prédéterminée (7) en transmettant le couple, et l'ampoule (2) étant réalisée de telle sorte que l'élément de rupture prédéterminée (7) se sépare de l'ampoule (2) au moins par sections sous l'effet d'une contrainte de couple déterminée exercée au moyen de l'élément d'actionnement (4),
**caractérisé en ce que** l'ampoule (2) est fabriquée en matière plastique (K) et présente une paroi (11) réalisée à plusieurs couches au moins par sections, et **en ce que** l'élément de rupture prédéterminée est formé d'un seul tenant sur l'ampoule (2) à une extrémité distale (8) de l'ampoule (2) sous la forme d'un bouton (7), le bouton (7) formant un rétrécissement à l'extrémité frontale d'une section transversale de l'ampoule (2), et le bouton (7) étant assemblé par complémentarité de forme dans la direction circonférentielle de l'ampoule (2) avec une section de réception complémentaire (8) du corps de réception (3), de telle sorte que l'ampoule (2) - du moins dans un état intact du bouton (7) - est reliée au corps de réception (3) dans la zone du bouton (7) de manière résistante au couple.

2. Agencement d'application médical (1) selon la revendication 1, **caractérisé en ce que** la paroi (11) présente au moins une couche barrière (12) contenant notamment un copolymère d'éthylène-alcool vinylique ou de l'alcool polyvinylique.

3. Agencement d'application médical (1) selon la revendication 1 ou 2, **caractérisé en ce que** la paroi (11) présente au moins une couche support (13) contenant notamment du polyéthylène ou du polypropylène.

4. Agencement d'application médical (1) selon l'une quelconque des revendications précédentes, **caractérisé en ce que** l'ampoule (2) est réalisée par coextrusion de matière plastique.

5. Agencement d'application médical (1) selon l'une quelconque des revendications précédentes, **caractérisé en ce que** l'ampoule (2) est réalisée de manière aseptique au moyen d'un procédé Blow-Fill-Seal.

6. Agencement d'application médical (1) selon l'une quelconque des revendications précédentes, **caractérisé en ce que** l'ampoule (2) présente au moins une section de paroi (14) conçue sous forme élastiquement souple.

7. Agencement d'application médical (1) selon l'une quelconque des revendications précédentes, **caractérisé en ce que** le corps de réception (3), l'élément d'application (5) et l'élément d'actionnement (4) sont fabriqués en matière plastique.

8. Agencement d'application médical (1) selon l'une quelconque des revendications précédentes, **caractérisé en ce que** l'élément de dosage (16) présente une membrane ayant l'élasticité du caoutchouc (17) qui est réalisée sous forme perméable en fonction de la pression.

9. Agencement d'application médical (1) selon la revendication 8, **caractérisé en ce que** la membrane (17) présente au moins une fente (18).

10. Agencement d'application médical (1) selon l'une quelconque des revendications précédentes, **caractérisé en ce que** le bouton (7) est conçu pour pouvoir se rompre de manière ductile.

11. Application d'application médical (1) selon l'une quelconque des revendications précédentes, **caractérisé en ce que** l'élément d'actionnement est réalisé, de préférence à une extrémité proximale (9) de l'ampoule (2), sous la forme d'un élément d'actionnement rotatif (4).
